(19) Europäisches Patentamt
European Patent Office
Office européen des brevets

(11) EP 1 066 821 A1

(12) **EUROPÄISCHE PATENTANMELDUNG**

(43) Veröffentlichungstag:
**10.01.2001 Patentblatt 2001/02**

(51) Int. Cl.$^7$: **A61K 7/06**, A61K 7/48,
A61K 7/42

(21) Anmeldenummer: **00113064.0**

(22) Anmeldetag: **26.06.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **08.07.1999 DE 19931707**
**26.01.2000 DE 10003234**

(71) Anmelder: **HAARMANN & REIMER GMBH**
**37601 Holzminden (DE)**

(72) Erfinder:
- **Ley, Jakob, Peter, Dr.**
  **37603 Holzminden (DE)**
- **Johncock, William, Dr.**
  **37471 Höxter (DE)**

(74) Vertreter:
**Mann, Volker, Dr. et al**
**Bayer AG**
**Konzernzentrale RP**
**Patente und Lizenzen**
**D-51368 Leverkusen (DE)**

(54) **Topische kosmetische Mittel, enthaltend Benzaldoxime**

(57) Benzaldoxime mit mindestens einer aromatischen Hydroxy- oder Alkoxygruppe sind wirksame Bestandteile in topischen kosmetischen Mittel, insbesondere Hautaufhellungsmittel.



Printed by Xerox (UK) Business Services
2.16.7 (HRS)/3.6

## Beschreibung

Die Erfindung betrifft topische kosmetische Mittel, insbesondere Hautaufhellungsmittel, enthaltend Benzaldoxime mit mindestens einer aromatischen Hydroxy- oder Alkoxygruppe, für kosmetische oder dermatologische Anwendungen.

Hautaufhellende Wirkstoffe greifen in irgendeiner Form in den Melaninmetabolismus bzw. -katabolismus ein. Die in der Regel braun bis schwarz gefärbten Melanine werden in den Melanocyten der Haut gebildet, in die Keratinozyten übertragen und verursachen die Färbung der Haut oder der Haare. Die braun-schwarzen Eumelanine werden in Säugetieren hauptsächlich aus hydroxysubstituierten aromatischen Aminosäuren wie L-Tyrosin und L-DOPA, die gelben bis roten Phäomelanine zusätzlich aus schwefelhaltigen Molekülen gebildet (*Cosmetics & Toiletries* **1996**, *111* (5), 43-51). Ausgehend von L-Tyrosin wird durch das kupferhaltige Schlüsselenzym Tyrosinase L-3,4-Dihydroxyphenylalanin (L-DOPA) gebildet, welches seinerseits wieder durch die Tyrosinase über das rot-braun gefärbte Dopachinon zu Melanin oxidiert wird. Ein Vergleich von Tyrosinasen aus Pflanzen, Pilzen und Säugetierzellen zeigt, dass der Mechanismus und die Substratspezifität bei allen untersuchten Tyrosinasen vergleichbar ist.

Sind die Melanin-bildenden Melanocyten in der menschlichen Haut aus irgendeinem Grund nicht gleichmäßig verteilt, kommt es zu Pigmentflecken, die entweder heller oder dunkler als die umgebenden Hautareale sind. Um dieses Problem zu beheben, werden auf dem Markt Hautaufhellungsmittel angeboten, die solche Pigmentflecken zumindest teilweise auszugleichen helfen. Zudem besteht für viele Menschen das Bedürfnis, ihre natürlicherweise dunkle Hautfarbe aufzuhellen. Dafür sind sehr sichere und wirkungsvolle Hautaufhellungsmittel notwendig. Viele Hautaufhellungsmittel enthalten mehr oder minder starke Tyrosinase-Inhibitoren.

In handelsüblichen Hautaufhellungsmitteln werden insbesondere Hydrochinon, Hydrochinonderivate wie z.B. Arbutin, Vitamin C, Derivate der Ascorbinsäure wie z.B. Ascorbylpalmitat, Kojisäure und Derivate der Kojisäure wie z.B. Kojisäuredipalmitat verwendet.

Eines der am häufigsten verwendeten Hautaufhellungsmittel ist Hydrochinon. Die Substanz hat aber einen cytotoxischen Effekt gegen Melanozyten und kann die Haut schädigen. Daher sind solche Präparate z.B. in Japan und Südafrika für kosmetische Anwendungen nicht mehr zulässig. Zudem ist Hydrochinon sehr oxidationsempfindlich und nur schwierig in kosmetischen Formulierungen zu stabilisieren.

Vitamin C und Ascorbinsäurederivate haben nur eine unzureichende Wirkung auf der Haut. Sie wirken zudem nicht direkt als Tyrosinase-Hemmstoffe, sondern reduzieren die farbigen Zwischenstufen der Melaninbiosynthese.

Kojisäure (5-Hydroxy-2-hydroxymethyl-4-pyranon) ist ein Tyrosinaseinhibitor, der über eine Chelatisierung der Kupferatome des Enzyms dessen katalytische Wirkung hemmt; sie wird in kommerziellen Hautaufhellungsmitteln eingesetzt. Die Substanz wird v.a. in Aspergillus-Kulturen gebildet und kann daraus nur in kleinen Mengen isoliert werden.

Die Aufgabe der vorliegenden Erfindung lag darin, preiswerte, einfach herzustellende, hochwirksame Tyrosinase-Inhibitoren zu finden, die als Wirkstoffe in Hautaufhellungsmitteln verwendet werden können.

Die Erfindung betrifft topische kosmetische Mittel, enthaltend Benzaldoxime der allgemeinen Formel

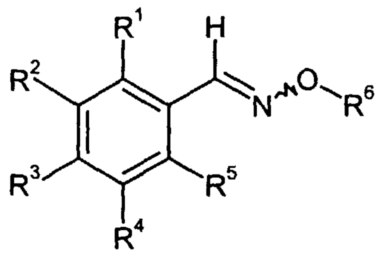

deren geometrischen Isomere oder Gemische dieser Isomere
wobei

| | |
|---|---|
| $R^1$, $R^5$ | unabhängig voneinander Wasserstoffatome oder Alkylgruppen mit 1 bis 4 C-Atomen darstellen, |
| $R^2$, $R^3$ und $R^4$ | unabhängig voneinander Wasserstoffatome, Hydroxygruppen, Alkylgruppen mit 1 bis 4 Kohlenstoffatomen oder Gruppen -O-$R^7$ darstellen, wobei $R^7$ eine Alkylgruppe mit 1 bis 4 Kohlenstoffatomen oder eine Arylalkylgruppe mit 7 bis 10 Kohlenstoffatomen darstellen kann, |

mit der Maßgabe, dass mindestens einer der Reste $R^2$ bis $R^4$ eine Hydroxygruppe oder eine Gruppe -O-$R^7$ darstellt,

wobei $R^7$ die oben angeführte Bedeutung hat,
und

$R^6$ ein Wasserstoffatom, eine Alkyl- oder Alkenylgruppe mit 1 bis 12 Kohlenstoffatomen oder eine gegebenenfalls substituierte Aryl- oder Arylalkylgruppe mit 7 bis 10 Kohlenstoffatomen oder eine gegebenenfalls substituierte Heteroaryl- oder Heteroarylalkylgruppe mit 2 bis 10 Kohlenstoffatomen, die ein oder mehrere Heteroatome aus der Gruppe Schwefel, Stickstoff oder Sauerstoff enthält, darstellt.

Bevorzugt sind topische kosmetische Mittel, enthaltend Benzaldoxime der allgemeinen Formel

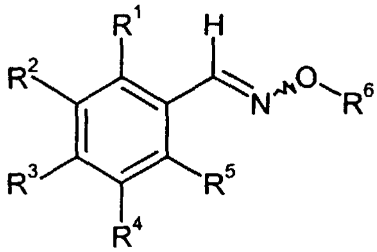

deren geometrischen Isomere oder Gemische dieser Isomere, wobei

$R^1$ und $R^5$ Wasserstoff bedeuten,
und

$R^2$, $R^3$ und $R^4$ unabhängig voneinander Wasserstoffatome, Hydroxygruppen, Alkylgruppen mit 1 bis 4 Kohlenstoffatomen oder Gruppen -O-$R^7$ darstellen, wobei $R^7$ eine Alkylgruppe mit 1 bis 4 Kohlenstoffatomen oder eine Arylalkylgruppe mit 7 bis 10 Kohlenstoffatomen darstellen kann,

mit der Maßgabe, dass mindestens einer der Reste $R^2$ bis $R^4$ eine Hydroxygruppe oder eine Gruppe -O-$R^7$ darstellt, wobei $R^7$ die oben angeführte Bedeutung hat,
und

$R^6$ ein Wasserstoffatom, eine Alkyl- oder Alkenylgruppe mit 1 bis 12 Kohlenstoffatomen oder eine gegebenenfalls substituierte Arylalkylgruppe mit 7 bis 10 Kohlenstoffatomen darstellt.

Insbesondere bevorzugt sind topische kosmetische Mittel, enthaltend Benzaldoxime ausgewählt aus der Gruppe umfassend z.B.

4-Hydroxybenzaldoxim
3,4,5-Trihydroxybenzaldoxim
3-Hydroxy-4-methoxybenzaldehyd-O-ethyloxim
3-Ethoxy-4-hydroxybenzaldehyd-O-ethyloxim
3,4-Dihydroxybenzaldoxim
3-Hydroxy-4-methoxybenzaldoxim
4-Hydroxy-3-methoxybenzaldehyd-O-ethyloxim
3,4-Dihydroxybenzaldehyd-O-(4-methylbenzyl)-oxim
3-Ethoxy-4-hydroxybenzaldoxim
4-Hydroxy-3-methoxybenzaldoxim
3,4-Dihydroxybenzaldehyd-O-ethyloxim

sind jedoch nicht darauf beschränkt.

Überraschenderweise wurde nun gefunden, dass die in den erfindungsgemäßen topischen kosmetischen Mitteln enthaltenden Benzaldoxime besonders starke Hemmstoffe der Tyrosinase sind. Insbesondere sind viele der erfindungsgemäßen Benzaldoxime wesentlich wirksamer als Kojisäure. Daher können sie als Wirkstoffe in kosmetischen oder dermatologischen Hautaufhellungsmitteln verwendet werden.

Die erfindungsgemäßen topischen kosmetischen Mittel, insbesondere Hautaufhellungsmittel, enthaltend

die Benzaldoxime, werden mit üblichen, an sich bekannten Verfahren hergestellt, dergestalt, dass eins oder mehrere der erfindungsgemäßen Benzaldoxime in kosmetische oder dermatologische Formulierungen eingearbeitet werden, die wie üblich zusammengesetzt sind und neben der hautaufhellenden Wirkung auch zur Behandlung, der Pflege und der Reinigung der Haut oder der Haare und als Schminkprodukte in der dekorativen Kosmetik dienen können.

Dementsprechend betrifft die vorliegende Erfindung auch topische kosmetische Mittel, insbesondere kosmetische und dermatologische Hautaufhellungsmittel, welche die erfindungsgemäßen Benzaldoxime in einer wirksamen Menge, nebst anderen, ansonsten üblichen Zusammensetzungsbestandteilen, umfassen. Sie enthalten 0,01 Gew.-% bis 30 Gew.-%, bevorzugt 0,01 bis 20 Gew.-%, insbesondere aber 0,01 Gew.-% bis 5 Gew.-%, bezogen auf das Gesamtgewicht der Formulierung, an den erfindungsgemäßen Benzaldoximen und können dabei als „Wasser in Öl"-, „Öl in Wasser"-, „Wasser in Öl in Wasser"- oder „Öl in Wasser in Öl"-Emulsionen, als Mikroemulsionen, als Gele, als Lösungen z.B. in Ölen, Alkoholen oder Siliconölen, als Stifte, als Seifen, als Aerosole, Sprays oder auch Schäume vorliegen. Weitere übliche kosmetische Hilfs- und Zusatzstoffe können in Mengen von 5 - 99 Gew.-%, vorzugsweise 10 - 80 Gew.-%, bezogen auf das Gesamtgewicht der Formulierung, enthalten sein. Ferner können die Formulierungen Wasser in einer Menge bis zu 99,99 Gew.-%, vorzugsweise 5- 80 Gew.-%, bezogen auf das Gesamtgewicht der Formulierung, aufweisen.

Die in den erfindungsgemäßen Hautaufhellungsmitteln enthaltenen Benzaldoxime sind teilweise bekannt. Die bekannten erfindungsgemäßen Benzaldoxime werden zum Beispiel in Chem. Ber. 1883, 16, 1780 bis 1787, Chem. Ber. 1941, 74, 79, 87 und 89, Chem. Ber. 1922, 55, 920 bis 929, in Chem. Ber. 1922, 55, 2357 bis 2372 und in Liebigs Ann. 1936, 526, 277 bis 294 beschrieben. Hinweise auf eine Wirkung als Tyrosinase-Inhibitoren und ihre Verwendung in kosmetischen und/oder dermatologischen Zubereitungen sind nicht gegeben. Die in den Hautaufhellungsmitteln enthaltenen erfindungsgemäßen Benzaldoxime werden, soweit noch nicht bekannt, nach den dem Fachmann bekannten Verfahren durch Umsetzung der entsprechenden Benzaldehyde mit den entsprechenden Hydroxylaminen oder deren Salzen in einem Lösungsmittel, bevorzugt in Wasser, einem aliphatischen Alkohol mit 1 bis 4 Kohlenstoffatomen oder einem Gemisch der Lösungsmittel, mit einer Hilfsbase, bevorzugt Natriumhydroxid oder Natriumacetat, bei 0°C bis 120°C, bevorzugt 20°C bis 100°C hergestellt, gegebenenfalls mit einer mineralischen Säure neutralisiert und mit den üblichen Verfahren, bevorzugt durch Kristallisation, gereinigt.

Als Benzaldehyde werden bevorzugt 4-Hydroxybenzaldehyd, 3,4,5-Trihydroxybenzaldehyd (Gallusaldehyd), 4-Methoxy-3-hydroxybenzaldehyd (Isovanillin), 3-Ethoxy-4-hydroxybenzaldehyd (Ethylvanillin), 3,4-Dihydroxybenzaldehyd (Protocatechualdehyd) oder 4-Hydroxy-3-methoxybenzaldehyd (Vanillin) verwendet.

Als Hydroxylamine werden bevorzugt Hydroxylamin, O-Ethylhydroxylamin oder O-4-Methylbenzylhydroxylamin oder die Salze der genannten Hydroxylamine verwendet.

Die erfindungsgemäßen topischen kosmetischen Mittel, insbesondere Hautaufhellungsmittel, können kosmetische Hilfs- und Zusatzstoffe enthalten, wie sie üblicherweise in solchen Zubereitungen verwendet werden, z.B. Sonnenschutzmittel (z.B. organische oder anorganische Lichtfiltersubstanzen, bevorzugt Mikropigmente), Konservierungsmittel, Bakterizide, Fungizide, Viruzide, Kühlwirkstoffe, Pflanzenextrakte, entzündungshemmende Wirkstoffe, die Wundheilung beschleunigende Stoffe (z.B. Chitin oder Chitosan und dessen Derivate), filmbildende Substanzen (z.B. Polyvinylpyrrolidone oder Chitosan oder dessen Derivate), gebräuchliche Antioxidantien, Vitamine (z.B. Vitamin C und Derivate, Tocopherole und Derivate, Vitamin A und Derivate), 2-Hydroxycarbonsäuren (z.B. Citronensäure, Apfelsäure, L-, D- oder dl-Milchsäure), Hautaufhellungsmittel (z.B. Kojisäure, Hydrochinon oder Arbutin), Hautfärbungsmittel (z.B. Walnussextrakte oder Dihydroxyaceton), Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen (z.B. Glycerin oder Harnstoff), Fette, Öle, ungesättigte Fettsäuren oder deren Derivate (z.B. Linolsäure, $\alpha$-Linolensäure, $\gamma$-Linolensäure oder Arachidonsäure und deren jeweiligen natürlichen oder synthetischen Ester), Wachse oder andere übliche Bestandteile einer kosmetischen oder dermatologischen Formulierung wie Alkohole, Polyole, Polymere, Schaumstabilisatoren, Elektrolyte, organische Lösungsmittel, Silikonderivate oder Chelatbildner (z.B. Ethylendiamintetraessigsäure und Derivate).

Die jeweils einzusetzenden Mengen an kosmetischen oder dermatologischen Hilfs- und Zusatzstoffen und Parfüm können in Abhängigkeit von der Art des jeweiligen Produkts vom Fachmann durch einfaches Ausprobieren leicht ermittelt werden.

Bevorzugt können die erfindungsgemäßen Hautaufhellungsmittel, enthaltend die erfindungsgemäßen Benzaldoxime auch andere Wirkstoffe zur Hautaufhellung enthalten. Insbesondere können die erfindungsgemäßen Hautaufhellungsmittel auch Kojisäure, Kojisäurederivate, Ascorbinsäure, Ascorbinsäurederivate, Hydrochinon, Hydrochinonderivate, schwefelhaltigen Moleküle wie z.B. Glutathion oder Cystein oder andere synthetische oder natürliche Wirkstoffe zur Hautaufhellung enthalten, wobei letztere auch in Form eines Extrakts aus Pflanzen, wie z.B. Bearberry-Extrakt und Reis-Extrakt verwendet werden können.

Die Menge der vorgenannten beispielhaften anderen Wirkstoffe zur Hautaufhellung (eine oder mehrere Verbindungen), welche nicht mit den in den erfindungsgemäßen Hautaufhellungsmitteln enthaltenen Benzaldoximen identisch sind, kann in den erfindungsgemäßen Hautaufhellungsmitteln 0,01 bis 30 Gew.-%, bevorzugt 0,01 bis 20 Gew-%,

besonders bevorzugt 0,01 bis 5 Gew.-%, bezogen auf das Gesamtgewicht der Zubereitung, betragen.

Die erfindungsgemäßen Hautaufhellungsmittel, enthaltend die erfindungsgemäßen Benzaldoxime können aber auch zusätzlich UVA- und/oder UVB-Filtersubstanzen enthalten, wobei die Gesamtmenge an Filtersubstanzen 0,1 bis 30 Gew.-%, vorzugsweise 0,5 bis 10 Gew.-%, bezogen auf das Gesamtgewicht der Zubereitungen, betragen kann, wobei man beispielsweise Sonnenschutzmittel für Haut und Haar erhält. Als UV-Filtersubstanzen können beispielsweise 3-Benzylidencampherderivate (z.B. 3-(4-Methylbenzyliden)-dl-campher), Aminobenzoesäurederivate (z.B. 4-(N,N-Dimethylamino)benzoesäure-2-ethylhexylester oder Methylanthranilat), 4-Methoxycinnamate (z.B. 2-Ethylhexyl-p-methoxycinnamat oder Isoamyl-p-methoxycinnamat), Benzophenone (z.B. 2-Hydroxy-4-methoxybenzophenon), ein- oder mehrfach sulfonierte UV-Filter [z.B. 2-Phenylbenzimidazol-5-sulfonsäure, Sulisobenzone oder 1,4-Bis(benzimidazolyl)-benzol-4,4',6,6'-tetrasulfonsäure bzw. 3,3'-(1,4-Phenylendimethyliden)-bis-(7,7-dimethyl-2-oxo-bicyclo-[2,2,1]heptan-1-methansulfonsäure) und deren Salze], Salicylate (z.B. 2-Ethylhexylsalicylat oder Homomethylsalicylat), Triazine {z.B. 2,4-Bis-[4-(2-ethylhexyloxy)-2-hydroxyphenyl]-6-(4-methoxyphenyl)-1,3,5-triazin, 4,4'-([6-([(1,1-dimethylethyl)-aminocarbonyl]phenylamino)-1,3,5-triazin-2,4-diyl]dimino)bisbenzoesäurebis-(2-ethylhexyl)-ester)}, 2-Cyanopropensäurederivate (z.B. 2-Ethylhexyl-2-cyano-3,3-diphenyl-2-propenoat), Dibenzoylderivate (z.B. 4-tert.-Butyl-4'-methoxydibenzoylmethan), polymergebundene UV-Filter (z.B. Polymer von N-[2-(bzw. 4)-(2-Oxo-3-bornyliden)methyl]-benzylacrylamid) oder Pigmente (z.B. Titandioxide, Zirkondioxide, Eisenoxide, Siliciumdioxide, Manganoxide, Aluminiumoxide, Ceroxide oder Zinkoxide) verwendet werden.

Die Lipidphase in den erfindungsgemäßen topischen kosmetischen Mitteln kann vorteilhaft gewählt werden aus folgenden Substanzgruppen: Mineralöle (vorteilhaft Paraffinöl), Mineralwachse, Kohlenwasserstoffe (vorteilhaft Squalan oder Squalen), synthetische oder halbsynthetische Triglyceridöle (z.B. Triglyceride der Caprin- oder Caprylsäure), natürliche Öle (z.B. Rizinusöl, Olivenöl, Sonnennblumenöl, Sojaöl, Erdnussöl, Rapsöl, Mandelöl, Palmöl, Kokusöl, Palmkernöl, Borretschsamenöl und dergleichen mehr), natürliche Esteröle (z.B. Jojobaöl), synthetische Esteröle (bevorzugt Ester von gesättigten und/oder ungesättigten, linearen und/oder verzweigten Alkancarbonsäuren von 3 bis 30 C-Atomen mit gesättigten und/oder ungesättigten, linearen und/oder verzweigten Alkoholen mit 3 bis 30 C-Atomen und Ester von aromatischen Carbonsäuren mit gesättigten und/oder ungesättigten, linearen und/oder verzweigten Alkoholen mit 3 bis 30 C-Atomen, insbesondere ausgewählt aus der Gruppe Isopropylmyristat, Isopropylstearat, Isopropylpalmitat, Isopropyloleat, n-Butylstearat, n-Hexyllaurat, n-Decyllaurat, Isooctylstearat, Isononylstearat, Isononylisononanoat, 2-Ethylhexylpalmitat, 2-Ethylhexyllaurat, 2-Hexyldecylstearat, 2-Octyldecylpalmitat, Oleyloleat, Oleylerucat, Erucyloleat, Erucylerucat sowie synthetische oder natürliche Gemische solcher Ester), Fette, Wachse und andere natürliche und synthetische Fettkörper, vorzugsweise Ester von Fettalkoholen mit Alkoholen niedriger C-Zahl (z.B. mit Isopropanol, Propylenglycol oder Glycerin) oder Ester von Fettalkoholen mit Alkansäuren niedriger C-Zahl oder mit Fettsäuren, Alkylbenzoate (z.B. Gemische von n-Dodecyl-, n-Tridecyl-, n-Tetradecyl- und n-Pentadecylbenzoat) sowie cyclische oder lineare Silikonöle (wie z.B. Dimethylpolysiloxane, Diethylpolysiloxane, Diphenylpolysiloxane sowie Mischformen daraus).

Die wässrige Phase der erfindungsgemäßen topischen kosmetischen Mittel enthält gegebenenfalls vorteilhaft Alkohole, Diole oder Polyole niedriger C-Zahl, sowie deren Ether, vorzugsweise Ethanol, Isopropanol, Propylenglycol, Glycerin, Ethylenglycol, Ethylenglycolmonoethyl- oder -monobutylether, Propylenglycolmonomethylether-, -monoethyl- oder -monobutyl-ether, Diethylenglycolmonomethyl- oder monoethylether und analoge Produkte, ferner Alkohole niedriger C-Zahl, z.B. Ethanol, Isopropanol, 1,2-Propandiol, Glycerin, weiterhin $\alpha$- oder $\beta$-Hydroxysäuren, vorzugsweise Milchsäure, Zitronensäure oder Salicylsäure, daneben Emulgatoren, welche vorteilhaft ausgewählt werden können aus der Gruppe der ionischen, nichtionischen, polymeren, phosphathaltigen und zwitterionischen Emulgatoren, sowie insbesondere ein oder mehrere Verdickungsmittel, welches oder welche vorteilhaft ausgewählt werden können aus der Gruppe Siliciumdioxid, Aluminiumsilikate, wie z.B. Bentonite, Polysaccharide bzw. deren Derivate, z.B. Hyaluronsäure, Guarkernmehl, Xanthangummi, Hydroxypropylmethylcellulose oder Allulose-Derivate, besonders vorteilhaft aus der Gruppe der Polyacrylate, bevorzugt ein Polyacrylat aus der Gruppe der sogenannten Carbopole, jeweils einzeln oder in Kombination oder aus der Gruppe der Polyurethane.

Zur Anwendung werden die erfindungsgemäßen topischen kosmetischen Mittel, insbesondere die Hautaufhellungsmittel, enthaltend die erfindungsgemäßen Benzaldoxime für Kosmetika üblichen Weise auf die Haut und/oder die Haare in ausreichender Menge aufgebracht.

**<u>Beispiele</u>**

Die nachfolgenden Beispiele sollen die vorliegende Erfindung verdeutlichen, ohne sie einzuschränken.

**Beispiel 1**: „Öl in Wasser"-Emulsion

| Teil | Rohstoffname (Hersteller) | Chemische Bezeichnung | Gehalt in Gew.-% |
|---|---|---|---|
| A | Arlatone 983 S® (ICI) | Ether von Polyethylenglycol mit Glycerylmonostearat | 1,2 |
| | Brij 76® (ICI) | 3,6,9,12,15,18,21,24,27,30,33,36-Decaoxaoctatetracontan-1-ol | 1,2 |
| | Cutina MD® (Henkel) | Glycerylmonostearat | 3,5 |
| | Baysiloneöl M10® (GE Bayer) | Polydimethylsiloxan | 0,8 |
| | Eutanol G® (Henkel) | Octyldodecanol | 3,0 |
| | Paraffinöl 65 cp (Henry Lamotte) | Mineralöl | 8,0 |
| B | Wasser, dest. | | 49,6 |
| | Phenopip® (Nipa Laboratorien) | 2-Phenoxyethanol und 4-Hydroxybenzoesäuremethylester und 4-Hydroxybenzoesäureethylester und 4-Hydroxybenzoesäure propylester und 4-Hydroxybenzoesäurebutyl-ester | 0,5 |
| | Trilon BD® (BASF) | Dinatrium-EDTA | 0,1 |
| | 1,2-Propylenglycol | | 2,0 |
| | Glycerin 99 % | | 3,0 |
| | **4-Hydroxy-3-methoxybenzaldoxim** | | 0,2 |
| C | Wasser, dest. | | 25,0 |
| | Carbopol 2050® (B.F. Goodrich) | vernetztes Acrylsäure/$C_{10}$-$C_{30}$-Alkylacrylat-Polymer | 0,4 |
| | wäßrige Natriumhydroxidlsg., 10 % | | 1,2 |
| D | Parfümöl | | 0,3 |

Teil A wurde gemischt und auf 80°C erhitzt. Teil B wurde gemischt und auf 90°C erhitzt und unter Rühren zu Teil A gegeben. Für Teil C wurde Carbopol in Wasser sorgfältig dispergiert und mit Natronlauge neutralisiert (pH 6,9). Teil C wurde dann bei 60°C zur Mischung aus den Teilen A und B gegeben. Teil D wurde zu der Mischung aus den Teilen A, B, und C bei Raumtemperatur hinizugefügt.

**Beispiel 2**: „Wasser in Öl"-Emulsion mit UVA/B-Breitbandschutz

| Teil | Rohstoffname (Hersteller) | Chemische Bezeichnung | Gehalt in Gew.-% |
|---|---|---|---|
| A | Dehymuls PGPH® (Henkel) | Polyglycerol-2 Dipolyhydroxystearat | 3,0 |
| | Monomuls 90-O 18® (Henkel) | Glyceryloleat | 1,0 |
| | Permulgin 2550® (Koster Keunen Holland) | Bienenwachs | 1,0 |
| | Myritol 318® (Henkel) | Capryl-/Caprinsäuretriglyceride | 6,0 |
| | Witconol TN® (Witco) | $C_{12}$-$C_{15}$-Alkylbenzoat | 6,0 |
| | Cetiol SN® (Henkel) | Cetyl- und Stearylisononanoat | 5,0 |
| | Copherol 1250® (Henkel) | Tocopherolacetat | 1,0 |
| | Solbrol P® (Bayer) | 4-Hydroxybenzoesäurepropylester | 0,1 |
| | Neo Heliopan® AV (Haarmann & Reimer) | 2-Ethylhexyl-p-methoxycinnamat | 4,0 |
| | Neo Heliopan® E 1000 (Haarmann & Reimer) | Isoamyl-p-methoxycinnamat | 4,0 |
| | Neo Heliopan® MBC (Haarmann & Reimer) | 3-(4-Methylbenzyliden)-dl-campher | 2,0 |
| | Neo Heliopan® OS (Haarmann & Reimer) | 2-Ethylhexylsalicylat | 3,0 |
| | Octyltriazon | | 1,0 |
| | Zinkoxid neutral (Haarmann & Reimer) | | 7,0 |
| B | Wasser, dest. | | 39,8 |
| | Trilon BD® (BASF) | Dinatrium-EDTA | 0,1 |
| | Phenoxyethanol | | 0,7 |
| | Solbrol M (Bayer) | 4-Hydroxybenzoesäuremethylester | 0,2 |
| | Glycerin 99 % | | 4,0 |
| | Neo Heliopan® Hydro (Haarmann & Reimer), | 2-Phenylbenzimidazol-5-sulfonsäure | 10,0 |
| | 15 % als Natriumsalz Benzophenon-4 | | 0,5 |
| | **4-Hydroxy-3-methoxybenzaldoxim** | | 0,2 |
| C | Parfümöl | | 0,3 |
| | Bisabolol | | 0,1 |

Für Teil A wurden alle Substanzen bis auf das Zinkoxid auf 85°C erhitzt und das Zinkoxid in der Mischung sorgfältig dispergiert. Die Komponenten des Teils B wurde gemischt, auf 85°C erhitzt und unter Rühren zu Teil A gegeben. Zu der Mischung aus den Teilen A und B wurde Teil C zugegeben und anschließend die Mischung mit einem Dispergierwerkzeug homogenisiert.

**Beispiel 3**: „Öl in Wasser"-Emulsion mit UVA/B-Breitbandschutz

| Teil | Rohstoffname (Hersteller) | Chemische Bezeichnung | Gehalt in Gew.-% |
|---|---|---|---|
| A | Arlacel 165® (ICI) | Glycerylstearat und Polyethylenglycol 100-Stearat | 3,0 |
| | Emulgin B2® (Henkel) | Ceteareth-20 | 1,0 |
| | Lanette O® (Henkel) | Cetyl- und Stearylalkohol | 1,15 |
| | Myntol 318® (Henkel) | Capryl-/Caprinsäuretriglyceride | 5,0 |
| | Cetiol SN® (Henkel) | Cetyl- und Stearylisononanoat | 4,0 |
| | Abil 100® (Goldschmidt) | Polydimethylsiloxan | 1,0 |
| | Bentone Gel MIO® (Rheox) | Mineralöl und Quaternium-18-hectorit und Propylencarbonat | 3,0 |
| | Cutina CBS® (Henkel) | Glycerylstearat und Cetylalkohol und Stearylalkohol und Cetylpalmitat und Kokosglyceride | 2,0 |
| | Neo Heliopan® 303 (Haarmann & Reimer) | 2-Ethylhexyl-2-cyano-3,3-diphenyl-2-propenoat | 7,0 |
| | Neo Heliopan® BB (Haarmann & Reimer) | 2-Hydroxy-4-methoxybenzophenon | 1,0 |
| | Neo Heliopan® MA (Haarmann & Reimer) | Menthylanthranilat | 3,0 |
| | N,N-Dimethyl-4-aminobenzoesäure-2-ethylhexylester | | 3,0 |
| | Titandioxid mikrofein | | 5,0 |
| B | Wasser, dest. | | 55,65 |
| | Trilon BD® (BASF) | Dinatrium-EDTA | 0,1 |
| | Veegum ultra® (Vanderbilt) | Magnesiumaluminiumsulfat | 1,0 |
| | Natrosol 250 HHR (Aqualon) | Hydroxymethylcellulose | 0,3 |
| | Glycerin | | 3,0 |
| | Phenopip® (Nipa Laboratorien) | 2-Phenoxyethanol und 4-Hydroxybenzoesäuremethylester und 4-Hydroxybenzoesäureethylester und 4-Hydroxybenzoesäurepropylester und 4-Hydroxybenzoesäurebutyl-ester | 0,3 |
| | **4-Hydroxy-3-methoxybenzaldoxim** | | 0,2 |
| C | Parfümöl | | 0,3 |

Für Teil A wurden alle Substanzen bis auf das Titandioxid gemischt und auf 85°C erhitzt; in die Mischung wurde das Titandioxid sorgfältig eindispergiert. Für Teil B wurden bis auf Veegum und Natrosol alle Substanzen gemischt, auf 90°C erhitzt, Natrosol und Veegum eindispergiert und die Mischung unter Rühren zu Teil A gegeben. Zu der Mischung aus den Teilen A und B wurde Teil C zugegeben und anschließend die Mischung mit einem Dispergierwerkzeug homogenisiert.

**Beispiel 4**: „Öl in Wasser"-Emulsion mit UVA/B-Breitbandschutz

| Teil | Rohstoffname (Hersteller) | Chemische Bezeichnung | Gehalt in Gew.-% |
|---|---|---|---|
| A | Crodaphos MCA® (Croda) | Cetylphosphat | 1,50 |
| | Cutina MD® (Henkel) | Glycerylstearat | 2,0 |
| | Lanette 16® (Henkel) | Cetylalkohol | 1,2 |
| | Myritol 318® (Henkel) | Capryl-/Caprinsäuretriglyceride | 5,0 |
| | Cetiol SN® (Henkel) | Cetyl- und Stearylisononanoat | 5,0 |
| | Copherol 1250® (Henkel) | Tocopherolacetat | 0,5 |
| | Solbrol P® (Bayer) | 4-Hydroxybenzoesäurepropylester | 0,1 |
| | Abil 100® (Goldschmidt) | Polydimethylsiloxan | 0,3 |
| | Trilon BD® (BASF) | Dinatrium-EDTA | 0,1 |
| | Neo Heliopan® HMS (Haarmann & Reimer) | 3,3,5-Trimethylcyclohexylsalicylat | 5,0 |
| | Butylmethoxydibenzoylmethan | | 2,0 |
| B | Wasser, dest. | | 47,6 |
| | 1,3-Butylenglycol | | 3,0 |
| | Sobrol M® (Bayer) | 4-Hydroxybenzoesäuremethylester | 0,2 |
| | Phenoxyethanol | | 0,7 |
| | Carbopol ETD 2050® (B.F. Goodrich) | Copolymer Acrylsäure/$C_{10}$-$C_{30}$-Alkylacrylat | 0,2 |
| | Keltrol T® (Calgon) | Xanthan-Gummi | 0,2 |
| | Neo Heliopan® AP (Haarmann & Reimer) | 2,2-(1,4-Phenylen)-bis-(1H-benzimidazol-4,6-disulfonsäure) und Dinatriumsalz | 22 |
| | **4-Hydroxy-3-methoxybenzaldoxim** | | 0,2 |
| C | wäßr. Natriumhydroxidlsg., 10 % | | 2,8 |
| D | Parfümöl | | 0,3 |
| | Bisabolol | | 0,1 |

Teil A wurde auf 85°C erhitzt. Carbopol und Keltrol wurden in die restlichen Bestandteile kalt eindispergiert, die Mischung auf 85°C erwärmt und zu Teil A gegeben. Teil C wurde sofort bei 80°C zu der Mischung aus den Teilen A und B gegeben und 5 min mit einem Dispergierwerkzeug homogensiert. Teil D wurde schließlich bei Raumtemperatur zugegeben und die Mischung mit einem Dispergierwerkzeug homogenisiert.

**Synthesevorschrift für die Benzaldoxime**

Der Benzaldehyd (87 mmol) wurde in 45 ml Wasser bei 40°C gelöst. Es wurde eine Lösung des entsprechenden Hydroxylaminhydrochlorids (90 mmol) und von Natriumacetat (87 mmol) in 25 ml Wasser zugegeben und die Reaktionsmischung bei ca. 80 °C 2 h unter Stickstoff gerührt. Die Mischung wurde nach dem Abkühlen mit 200 ml tert-Butylmethylether extrahiert, die organische Phase mit gesättigter Natriumchloridlösung gewaschen, über Natriumsulfat getrocknet, abfiltriert und das Filtrat im Vakuum zur Trockene eingedampft. Der kristalline Rückstand wurde gegebenenfalls umkristallisiert.

Tabelle 1

| Testverbin dung | **Name** | **CAS-Nr.** | **Smp./°C** | MS |
|---|---|---|---|---|
| 1 | 4-Hydroxybenzaldoxim | 699-06-9 | 86,3 | EI: *m/z* = 137 (100 %), 120 (14 %), 119 (16 %), 94 (71 %), 93 (21 %), 65 (39 %), 64 (12 %), 63 (15 %), 53 (10 %), 39 (21 %) |
| 2 | 3,4,5-Trihydroxybenzaldoxim | 53148-14-4 | 177 (Zers.) | APCI-(+): *m/z* = 170,0 (100 %), 154,3 (11 %) |
| 3 | 3-Hydroxy-4-methoxybenzaldehyd-O-ethyloxim | - | 61,4 | EI: *m/z* = 195 (199 %), 140 (20 %), 124 (36 %), 123 (27 %), 106 (21 %), 79 (22 %), 65 (22 %), 52 (25 %), 51 (22 %), 29 (27 %) |
| 4 | 3-Ethoxy-4-hydroxybenzaldehyd-O-ethyloxim | - | 43 | EI: *m/z* = 209 (100 %), 153 (39 %), 152 (25 %), 136 (26 %), 126 (28 %), 110 (36 %), 52 (23 %), 51 (20 %), 29 (40 %), 27 (26 %) |
| 5 | 3,4-Dihydroxybenzaldoxim | 3343-59-7 | 143 (Zers.) | ESI-(-): *m/z* = 305,0 (100 %), 152,2 (80 %) |
| 6 | 3-Hydroxy-4-methoxybenzaldoxim | 51673-94-0 | 142,4 | EI: *m/z* = 167 (100 %), 152 (31 %), 134 (14 %), 125 (14 %), 124 (52 %), 109 (15 %), 106 (16 %), 79 (20 %), 52 (19 %), 51 (21 %) |
| 7 | 4-Hydroxy-3-methoxybenzaldehyd-O-ethyloxim | - | 31,7 | EI: *m/z* = 195 (100 %), 167 (21 %), 150 (19 %), 134 (16 %), 125 (20 %), 124 (25 %), 123 (28 %), 106 (16 %), 52 (18 %), 29 (17 %) |
| 8 | 3,4-Dihydroxybenzaldehyd-O-(4-methylbenzyl)-oxim | - | 85-86 | APCI-(-): *m/z* = 256,3 (100 %) |
| 9 | 3-Ethoxy-4-hydroxybenzaldoxim | 52005-82-0 | 189 (Zers.) | EI: *m/z* = 181 (90 %), 153 (28 %), 152 (17 %), 136 (26 %), 135 (38 %), 126 (21 %), 110 (100 %), 52 (19 %), 51 (18 %), 29 (16 %) |
| 10 | 4-Hydroxy-3-methoxybenzaldoxim | 2874-33-1 | 118,2 | EI: *m/z* = 167 (100 %), 152 (13 %), 134 (22 %), 125 (21 %), 124 (61 %), 109 (18 %), 106 (19 %), 79 (15 %), 52 (15 %), 51 (16 %) |
| 11 | 3,4-Dihydroxy-benzaldehyd-O-ethyloxim | - | flüssig bei 23°C | EI: *m/z* = 181 (100 %), 153 (20 %), 152 (19 %), 136 (28 %), 126 (26 %), 110 (47 %), 109 (30 %), 81 (18 %), 53 (14 %), 29 (16 %) |

**Experiment**

Die Aktivität zur Hemmung der Tyrosinase der Testverbindungen 1 bis 11 wurde mit der der Kojisäure wie folgt verglichen:

Das aus Pilzen extrahierte Enzym Tyrosinase wurde von der Firma Sigma-Aldrich bezogen. Die Tyrosinase (2000 Einheiten/mg) wurde in Phosphatpuffer (pH 6,8, 0,067 mol/l) zu einer Konzentration von 120 Einheiten/ml gelöst und jeweils 100 µl dieser Tyrosinase-Lösung wurden in eine Kavität einer Mikrotiterplatte aus Polystyrol gegeben. 25 µl Phosphatpuffer (pH 6,8, 0,067 mol/l) und 75 µl von schrittweise verdünnter Testverbindung 1 bis 11 oder Kojisäure wurden hinzugegeben. Die sich ergebende Mischungen wurden 10 min bei 37°C inkubiert. Zur Verdünnung der Testverbindungen wurde Phosphatpuffer (pH 6,8, 0,067 mol/l) verwendet. Als Kontrolle wurde Phosphatpuffer (pH 6,8, 0,067 mol/l) verwendet.

100 µl einer 0,03 %igen Lösung des Substrats L-DOPA in Phosphatpuffer (pH 6,8, 0,067 mol/l) wurden hinzugegeben und die Absorption (A) bei 475 nm mit Hilfe eines Photometers nach 3 min Inkubation bei 37°C gemessen. Die Restaktivitäten der Tyrosinase in Anwesenheit der Beispiele 1 bis 11 oder der Kojisäure wurden gemäß der folgenden Gleichung berechnet:

$$\text{Restaktivität der Tyrosinase (\%)} = (A_{\text{Testverbindung}}/A_{\text{Kontrolle}}) \times 100$$

Aus den Restaktivitäten (%) der Tyrosinase in einer Reihe von Verdünnungen von Testverbindungen wurde für jede Testverbindung die $HK_{50}$ berechnet. Dies ist die Konzentration einer Testverbindung, bei der die Tyrosinase zu 50 % gehemmt wird.

Tabelle 2

| | **$HK_{50}$ (µM)** |
|---|---|
| Kojisäure | 22 |
| Testverbindung 1 | 25 |
| Testverbindung 2 | 20 |
| Testverbindung 3 | 18 |
| Testverbindung 4 | 14 |
| Testverbindung 5 | 18 |
| Testverbindung 6 | 4.6 |
| Testverbindung 7 | 4.2 |
| Testverbindung 8 | 3 |
| Testverbindung 9 | 3.5 |
| Testverbindung 10 | 2.3 |
| Testverbindung 11 | 0.3 |

**Patentansprüche**

**1.** Topische kosmetische Mittel, enthaltend Benzaldoxime der allgemeinen Formel

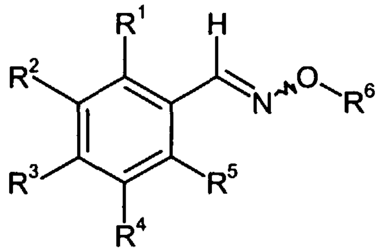

deren geometrischen Isomere oder Gemische dieser Isomere
wobei

$R^1$, $R^5$ unabhängig voneinander Wasserstoffatome oder Alkylgruppen mit 1 bis 4 C-Atomen darstellen,

$R^2$, $R^3$ und $R^4$ unabhängig voneinander Wasserstoffatome, Hydroxygruppen, Alkylgruppen mit 1 bis 4 Kohlenstoffatomen oder Gruppen -O-$R^7$ darstellen, wobei $R^7$ eine Alkylgruppe mit 1 bis 4 Kohlenstoffatomen oder eine Arylalkylgruppe mit 7 bis 10 Kohlenstoffatomen darstellen kann,

mit der Maßgabe, dass mindestens einer der Reste $R^2$ bis $R^4$ eine Hydroxygruppe oder eine Gruppe -O-$R^7$ darstellt, wobei $R^7$ die oben angeführte Bedeutung hat,
und

$R^6$ ein Wasserstoffatom, eine Alkyl- oder Alkenylgruppe mit 1 bis 12 Kohlenstoffatomen oder eine gegebenenfalls substituierte Aryl- oder Arylalkylgruppe mit 7 bis 10 Kohlenstoffatomen oder eine gegebenenfalls substituierte Heteroaryl- oder Heteroarylalkylgruppe mit 2 bis 10 Kohlenstoffatomen, die mehr als ein Heteroatom aus der Gruppe Schwefel, Stickstoff oder Sauerstoff enthält,

darstellt.

**2.** Topische kosmetische Mittel, enthaltend Benzaldoxime der allgemeinen Formel

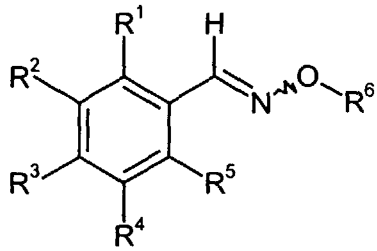

deren geometrischen Isomere oder Gemische dieser Isomere, wobei

$R^1$ und $R^5$ Wasserstoff bedeuten,
und

$R^2$, $R^3$, und $R^4$ unabhängig voneinander Wasserstoffatome, Hydroxygruppen, Alkylgruppen mit 1 bis 4 Kohlenstoffatomen oder Gruppen -O-$R^7$ darstellen, wobei $R^7$ eine Alkylgruppe mit 1 bis 4 Kohlenstoffatomen oder eine Arylalkylgruppe mit 7 bis 10 Kohlenstoffatomen darstellen kann,

mit der Maßgabe, dass mindestens einer der Reste $R^2$ bis $R^4$ eine Hydroxygruppe oder eine Gruppe -O-$R^7$ darstellt, wobei $R^7$ die oben angeführte Bedeutung hat,
und $R^6$ ein Wasserstoffatom, eine Alkyl- oder Alkenylgruppe mit 1 bis 12 Kohlenstoffatomen oder eine gegebenenfalls substituierte Arylalkylgruppe mit 7 bis 10 Kohlenstoffatomen darstellt.

**3.** Topische kosmetische Mittel, enthaltend

4-Hydroxybenzaldoxim
3,4,5-Trihydroxybenzaldoxim
3-Hydroxy-4-methoxybenzaldehyd-O-ethyloxim
3-Ethoxy-4-hydroxybenzaldehyd-O-ethyloxim
3,4-Dihydroxybenzaldoxim
3-Hydroxy-4-methoxybenzaldoxim
4-Hydroxy-3-methoxybenzaldehyd-O-ethyloxim
3,4-Dihydroxybenzaldehyd-O-(4-methylbenzyl)oxim
3-Ethoxy-4-hydroxybenzaldoxim
4-Hydroxy-3-methoxybenzaldoxim
3,4-Dihydroxybenzaldehyd-O-ethyloxim.

**4.** Topische kosmetische Mittel nach den Ansprüchen 1 bis 3, enthaltend 0,01 Gew.-% bis 30 Gew.-%, bevorzugt 0,01 bis 20 Gew.-%, insbesondere aber 0,01 Gew.-% bis 5 Gew.-%, bezogen auf das Gesamtgewicht der Formulierung, der Benzaldoxime der allgemeinen Formel I.

**5.** Verwendung der topischen kosmetischen Mittel nach Ansprüchen 1 bis 4 als Hautaufhellungsmittel.

**6.** Verwendung der Benzaldoxime nach den Ansprüchen 1 bis 3 in kosmetischen Zubereitungen.

**7.** Verwendung der Benzaldoxime nach den Ansprüchen 1 bis 3 in dermatologischen Zubereitungen.

**8.** Verwendung der Benzaldoxime nach den Ansprüchen 1 bis 3 in Sonnenschutzmitteln.

**9.** Verwendung der Benzaldoxime nach den Ansprüchen 1 bis 3 in Haarpflegeprodukten.

**10.** Verwendung der Benzaldoxime nach den Ansprüchen 1 bis 3 in Anti-Hautalterungsprodukten.

Europäisches Patentamt

# EUROPÄISCHER RECHERCHENBERICHT

Nummer der Anmeldung
EP 00 11 3064

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | WO 95 01157 A (PROCTER & GAMBLE) 12. Januar 1995 (1995-01-12)<br>* Ansprüche 1,16 *<br>----- | 1,4,6-8 | A61K7/06<br>A61K7/48<br>A61K7/42 |

| RECHERCHIERTE SACHGEBIETE (Int.Cl.7) |
|---|
| A61K |

Der vorliegende Recherchenbericht wurde für alle Patentansprüche erstellt

| Recherchenort | Abschlußdatum der Recherche | Prüfer |
|---|---|---|
| DEN HAAG | 10. November 2000 | Peeters, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 00 11 3064

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-11-2000

| Im Recherchenbericht angeführtes Patentdokument | Datum der Veröffentlichung | Mitglied(er) der Patentfamilie | Datum der Veröffentlichung |
|---|---|---|---|
| WO 9501157 A | 12-01-1995 | KEINE | |

EPO FORM P0461

Für nähere Einzelheiten zu diesem Anhang : siehe Amtsblatt des Europäischen Patentamts, Nr.12/82